(19) Europäisches Patentamt
European Patent Office
Office européen des brevets

(11) EP 2 785 196 B1

(12) **EUROPEAN PATENT SPECIFICATION**

(45) Date of publication and mention
of the grant of the patent:
**05.08.2020 Bulletin 2020/32**

(21) Application number: **12854533.2**

(22) Date of filing: **03.12.2012**

(51) Int Cl.:
*A23J 1/00* (2006.01)  *C07K 1/00* (2006.01)
*C12P 21/06* (2006.01)  *C07K 1/14* (2006.01)
*C07K 14/555* (2006.01)  *C07K 14/54* (2006.01)
*C07K 1/113* (2006.01)  *C12P 21/02* (2006.01)
*C12N 9/50* (2006.01)

(86) International application number:
**PCT/US2012/067608**

(87) International publication number:
**WO 2013/082599 (06.06.2013 Gazette 2013/23)**

(54) **METHODS AND SYSTEMS FOR PROTEIN REFOLDING**

VERFAHREN UND SYSTEME ZUR PROTEINRÜCKFALTUNG

PROCÉDÉS ET SYSTÈMES POUR LE REPLIEMENT D'UNE PROTÉINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.12.2011 US 201161565768 P**

(43) Date of publication of application:
**08.10.2014 Bulletin 2014/41**

(73) Proprietor: **Pressure BioSciences, Inc.
South Easton, MA 02375 (US)**

(72) Inventors:
• **SEEFELDT, Matthew
Aurora, Colorado 80045 (US)**
• **GOMEZ, Eliana
Aurora, Colorado 80045 (US)**

(74) Representative: **Grund, Martin
Grund Intellectual Property Group
Patentanwalt und Solicitor PartG mbB
Postfach 44 05 16
80754 München (DE)**

(56) References cited:
**WO-A1-00/02901        WO-A1-2009/035617
US-A1- 2009 076 247   US-A1- 2009 208 453
US-A1- 2009 208 453   US-A1- 2010 075 399**

• **AMBER COTHRAN, RICHARD J. ST. JOHN, CHARLES H. SCHMELZER, SHELLY A. PIZARRO: "High-Pressure Refolding of human Vascular Endothelial Growth Factor (VEGF) Recombinantly Expressed in Bacterial Inclusion Bodies: Refolding Optimization, and Feasibility Assessment", BIOTECHNOLOGY PROGRESS, vol. 27, no. 5, 23 May 2011 (2011-05-23), pages 1273-1281, XP002741124,**
• **ST JOHN R J ET AL: "High pressure fosters protein refolding from aggregates at high concentrations", PROCEEDINGS OF THE NATIONAL ACADEMY OF SCIENCES, NATIONAL ACADEMY OF SCIENCES, US, vol. 96, no. 23, 9 January 1999 (1999-01-09), pages 13029-13033, XP002227216, ISSN: 0027-8424, DOI: 10.1073/PNAS.96.23.13029**
• **SCHMOEGER E ET AL: "Matrix-assisted refolding of autoprotease fusion proteins on an ion exchange column", JOURNAL OF CHROMATOGRAPHY, ELSEVIER SCIENCE PUBLISHERS B.V, NL, vol. 1216, no. 48, 27 November 2009 (2009-11-27), pages 8460-8469, XP026740852, ISSN: 0021-9673, DOI: 10.1016/J.CHROMA.2009.10.012 [retrieved on 2009-10-12]**
• **BAROFOLD. CLIENT SERVICES 2010, XP055151200 Retrieved from the Internet: <URL:http://www.barofold.com/client-services.html> [retrieved on 2013-02-27]**

EP 2 785 196 B1

(Cont. next page)

- 'High Pressure Equipment Company' REACTOR GUIDE SELECTION, [Online] 19 November 2011, XP003031231 Retrieved from the Internet: <URL:http://web.archive.org/web/20111119202745/http://www.highpressure.com/products/reactors-pressure-vessels/reactor-selection-guide> [retrieved on 2013-02-27]

**Description**

FIELD OF THE INVENTION

The present disclosure relates to high pressure disaggregation and refolding of recombinant proteins from inclusion bodies, prepared as stabilized protein inclusion body preparations or dispersions, for large scale protein manufacturing.

BACKGROUND

While commercial production of recombinant protein in *E. coli* has many advantages, some proteins fold incorrectly in this system forming inclusion bodies (IB) that require solubilization and refolding to native conformation. This refolding process often represents a bottleneck, with the average yield of the protein being in the range of 15-25%. See Zhang et al., Modeling of protein refolding from inclusion bodies, Acta Biochim. Biophys Sin 1044-1052 (2009). Processes for increasing the yield of refolded protein from inclusion bodies are desired.

US 2009/208453 discloses a method for refolding inclusion bodies using high pressure treatment in a pressure vessel of 10 L volume.

SUMMARY OF THE INVENTION

Chemical denaturants, including chaotropes such as urea or guanidium chloride and including denaturing surfactants such as sodium dodecyl sulfate ("SDS"), have been traditionally used to solubilize and refold proteins from inclusion bodies, where high concentrations of these agents (e.g., up to 6M guanidine HCl, 8M urea, 0.1% SDS) thermodynamically denature the protein. Refolding is achieved by removing the chaotrope or detergent after inclusion body and/or aggregate dissociation, commonly via dilution, dialysis, or diafiltration. Inclusion body properties and size have negligible impact on refolding yield in these systems as they are dissolved and denatured at large-scale in mixing tanks prior to refolding.

High hydrostatic pressure is also a potentially effective protein refolding tool and is described in U.S. Patent. Nos. 7,064,192 and 6,489,450. In contrast to traditional chaotrope-based refolding, high pressure techniques can dissociate protein aggregates under conditions that favor the protein's native conformation, and can be conducted in the absence of chaotropes or strong-binding detergents, facilitating downstream purification. However, hydrostatic pressure is less demonstrated at large commercial scales, which can result in reduced or unacceptable yield in refolded protein from inclusion bodies, especially where the use of chaotropes and denaturing detergents are not preferred.

The present invention provides methods and systems for production of recombinant protein, and particularly, for production of recombinant protein from inclusion bodies. The invention is applicable at large scales, to support commercial production of recombinant proteins from inclusion bodies. For example, in one aspect, the method comprises providing a protein preparation comprising inclusion bodies, preparing a stable inclusion body preparation or dispersion, and exposing the inclusion body dispersion to high pressure in a pressure vessel, to thereby disaggregate and refold the recombinant protein to native (e.g., active) conformation.

The present invention employs mechanical shear and selection of solution chemistry to create the stable inclusion body preparation or dispersion. The inclusion body dispersion comprises inclusion bodies of a substantially homogeneous small size of less than 10 μm, so that the settling rate is less than about 5 cm per hour, or in some embodiments is less than about 1 cm per hour or the settling rate is negligible due to Brownian motion (that is, as a result of particles being less than about 2.2 μm). In this manner, inclusion bodies that are loaded into high pressure refolding reaction and pressure treated will have ample time to become disaggregated and solubilized prior to settling to the bottom of the pressure vessel. In various embodiments, the inclusion body preparation or dispersion is non-denaturing, that is, it does not contain a denaturing amount of detergents (e.g., SDS) or chaotropic agents (e.g., urea or guanidinium chloride), or is entirely free of such agents. The dispersion is created without solubilizing and precipitating the protein from inclusion bodies prior to high pressure treatment. In some embodiments, the dispersion is stable for at least about 1 week, at least about 2 weeks, or at least about 1 month, without substantial settling or need for redispersion processes before high pressure treatment. The preparation or dispersion is stable both with and without one or more steps of freeze/thaw. The invention therefore can provide for process efficiencies without loss, or with significant improvement in, refolding yield. The inclusion body dispersion allows for improved yield during high pressure refolding, including large scale refolding, by among other things, avoiding settling of inclusion bodies and/or reducing the aggregating propensity of the inclusion body particles during high pressure treatment.

In some aspects and embodiments, the size of the inclusion body particles, their settling rate, and/or aggregation propensity are adjusted by modifying the solution pH, or altering the solution dielectric constant, through the addition of a non-denaturing detergent, refolding additive, and/or viscosity-increasing agent.

In still other aspects and embodiments, the horizontal axis of the pressure vessel is at least twice the vertical axis. A pressure vessel with a large horizontal surface area supports greater yield through high pressure folding. Vertical loading high pressure vessels have generally been used for conducting high pressure experiments and food pasteurization.

Other aspects and embodiments of the invention will be apparent from the following detailed description.

DESCRIPTION OF THE FIGURES

FIGURE 1 shows the particle size distributions of four inclusion body preparations. All samples exhibit rapid settling as observed by visual inspection and as predicted by Stokes flow.

FIGURE 2 shows particle size distributions of rhG-CSF inclusion bodies using FlowCAM imaging. The results correlate with Coulter Counter analysis, specifically there are large amounts of particles in the size range of 5 to 50 $\mu$m.

FIGURE 3 shows settling distance of inclusion bodies as a function of particle size and determined by Stokes flow. Particles greater than 5 $\mu$m in diameter settle sufficient to result in a protein concentration gradient in a non-mixed high pressure refold after thirty minutes.

FIGURE 4 shows particle size distributions of four inclusion body preparations after high pressure homogenization. Samples were disperse and did not settle as observed by visual inspection and as predicted by Stokes flow and Equation 3.

FIGURE 5 shows inclusion body dispersion prior to refolding increases refolding yields significantly in a manner that is scale independent.

FIGURE 6 shows inclusion body high pressure homogenization of rhG-CSF inclusion bodies increases refolding yields and minimizes scale impacts.

FIGURE 7 shows the addition of 20% glycerol during Fab 1664 freeze/thaw at 20°C prevents flocculation.

FIGURE 8 shows the effect of homogenization techniques, in attempts to create dispersions, on particle size of Fab 1664.

DETAILED DESCRIPTION OF THE INVENTION

The invention provides methods and systems for production of recombinant protein, and particularly, for production of recombinant protein from inclusion bodies. For example, in one aspect, the method comprises providing a protein preparation comprising inclusion bodies, preparing a stable inclusion body preparation or dispersion, and exposing the inclusion body dispersion to high pressure in a pressure vessel, to disaggregate and refold the inclusion body protein. By preparing the stable inclusion body dispersion prior to high pressure treatment, the invention avoids the loss of refolding yield caused by large protein concentration gradients and limits in protein solubility, especially during large scale refolding.

The protein concentration within a refolding reaction impacts the refolding yield in at least two ways. First, detrimental reaggregation reactions are more prevalent if the refolding reaction is conducted at a high protein concentration, since the aggregation reaction order has been reported to be approximately 2.6, while the folding reaction is first order. Second, the maximum protein concentration that can be obtained in a solution is governed by the protein solubility. The solubility of a protein is a function of the characteristics of the given protein molecule and solution thermodynamics (e.g. pH, ionic strength, dielectric). If a refolding reaction is attempted at protein concentration that is above the solubility limit, the yield will be adversely affected by the precipitation of protein at the saturation concentration.

The fundamental effect of protein concentration on aggregation reactions has been observed empirically during high pressure refolds. For example, yields of soluble, monomeric rhGH (recombinant human growth hormone) from aggregates after a 24-hour incubation at 2000 bar were essentially independent of protein concentration in the range 0.87 to 8.7 mg/ml (St. John, Carpenter et al. 1999). However, other proteins have a higher sensitivity to protein concentration during high pressure refolding. Recombinant human placental bikunin exhibited refolding yields of 96% at protein concentrations of 0.0625 mg/ml, with the yield decreasing to 44% at a concentration of 2 mg/ml (Seefeldt, Ouyang et al. 2004). Lysozyme, refolded using high hydrostatic pressure, had yields of 80% and 65% at protein concentration of 0.25 and 2 mg/ml respectively (St. John, Carpenter et al. 1999). Protein concentration can therefore be an important variable in modulating high pressure refolding yields.

High pressure refolding chambers are conducted in a batch manner without the ability to agitate, which impacts the manner in with the technology can be applied to insoluble material, such as inclusion bodies. Inclusion bodies are formed during recombinant expression in *E. coli.* and are characterized as dense structures of misfolded expressed polypeptides that arise from the inability of cellular machinery to process and refold the polypeptide correctly. Inclusion bodies have been measured to be in the size range of about 0.5 to about 1 $\mu$m and can be harvested from bacterial cells using sonication or high pressure homogenizer. During processing, centrifugation, and storage, most inclusion body preparations will flocculate and agglomerate increasing their size significantly. The extent of flocculation is dependent on the amount cell debris (e.g., residual DNA), pH, and solution conditions of the harvesting solution. In one example, the zeta potential for an inclusion body preparation was observed to be about -9.8 mV (see US 2011/0268773). Values between -10 mV to +10 mV are generally thought to characterize an unstable solution with a high propensity to flocculate. Once flocculated, the inclusion bodies will settle to the bottom of the aqueous refolding solution, potentially causing a significant impact on the refolding yield, especially for large scale refolding reactions. This settling problem results in a protein concentration gradient and a large apparent protein concentration at regions of the pressure vessel. More specifically, the *apparent* protein concentration during the refold is much higher than the actual concentration of the sample, due to inclusion body settling. If there was infinite packing of the inclusion bodies, the protein concentration at the bottom of the vessel would be ~1260 mg/ml (based on the density of protein in inclusion bodies). Packing inefficiencies result in a protein concentration of inclusion body slurries that is typically in the range of 20-100 mg/ml. The time required for the formation of this high protein concentration regime is governed by Stokes flow (Eqn. 1) of the inclusion body particles, e.g., for particles above about 2.2 $\mu$m that are not governed by Brownian motion,

$$\text{Eqn. 1} \quad V = \frac{(D^2 \, g \, (\rho_{part} - \rho_{fluid}))}{18\mu}$$

where V is the velocity, D is the particle diameter, g is the gravitation constant, $\mu$ is the solution viscosity, and $\rho$ is the density of particle and fluid respectively (de Nevers 1970).

The average kinetic energy per molecule can be quantified by (Eqn. 2):

$$\text{Eqn. 2} \quad E_k = 1.5*k_b*T$$

where $E_k$ is the average kinetic energy per molecule, $k_b$ is the Boltzman constant, and T is the temperature (K) )(Laidler and Meiser 1995).

If one solves the energy balance on a particle from gravitational forces (Eqn 1) and kinetic forces due to Brownian motion (Eqn. 2), the following relationship can be developed (Eqn 3.)

$$\text{Eqn. 3} \quad \frac{D^4 \, g \, (\rho_{part} - \rho_{fluid}))}{16 K_b T} = \frac{Gravitational\ Forces}{Brownian\ Motion}$$

where values greater than 1 demonstrate that gravitation forces dominate and particle settling can be generally characterized by Stokes flow and with values less than 1 describing dispersion where settling no longer occurs due to overcoming forces associated with the kinetic motion of molecules (Duffy and Hill 2011). Solving equation 3 for approximately spherical inclusion bodies with a density of about 1.25g/ml results in the general approximation that particles greater than about 2.2 $\mu$m in diameter settle as described by Stokes flow and particles less than about 2.2 $\mu$m in diameter form colloids where settling does not occur, because the solution is dictated by Brownian motion. There are shape factors associated with the non-spherical nature of some inclusion bodies, resulting in a distribution of fluid dynamic properties around the parameters identified above.

Thus, in accordance with the invention, a stable dispersion of inclusion bodies is prepared. Upon high pressure treatment, the inclusion bodies do not settle thereby providing improved refolding yields and/or process efficiencies since solubilization with chaotropes is rendered unnecessary. The inclusion body dispersion may be prepared by, for example, high pressure homogenization or other technique involving high shear stress.

As used herein, the term "dispersion" or "stable protein preparation" are used interchangeably, and refer to a substantially aqueous system in which fine solid particles are uniformly dispersed. The preparation or dispersion is thus a two-phase liquid system where one phase consists of finely divided particles of insoluble inclusion bodies distributed throughout the second phase, which is substantially water. The insoluble inclusion bodies may be composed of at least two groups of particles. First, the dispersion may have particles that are less than about 2.2 $\mu$m in size, defined as a colloid, where settling does not substantially occur due to Brownian motion. Second, the dispersion may have particles greater than about 2.2 $\mu$m in size, but less than about 10 $\mu$m, or less than about 5 $\mu$m in certain embodiments, where settling occurs due to Stokes flow, but the rate is slow enough to improve high pressure refolding at large scales. The fine particles are substantially uniform in size, with the majority of inclusion body particles by mass having a size of less than about 5$\mu$m, and which do not substantially vary. For example, at least 75% or at least 85%, or at least 90%, or at least 95% of the inclusion body particles are with a 5$\mu$m size window. In some embodiments, at least 75%, or at least 85%, or at least 90%, or at least 95% of the inclusion body particles are within a 2μm size window. The small size and size distribution of the fine inclusion body particles renders the dispersion more stable from settling during high pressure treatment. In addition, in some embodiments, the inclusion body particles in the dispersion show a decreased propensity for aggregation, rendering the overall process more efficient, practical, and consistent from batch-to-batch. In some embodiments, the dispersion is further stabilized by the use of pH, ionic strength, preferential excluding compounds, detergents, and polymers which increase the stability of the dispersion by altering zeta potential. Thus, the inclusion body dispersion is designed to consist essentially of particles that are less than about 10 μm or less than about 5 μm in size to prevent settling or be small enough that settling rates are minimized as described by Stokes flow. In various embodiments, these particles have a zeta potential that is outside the range of ±10, ±20, or ±30 to avoid aggregation propensity.

As disclosed herein, the diameter of the inclusion body particles and their distribution, sufficient for guiding refold yield, can be quantified through the use of at least one of Coulter Counter, imaging, laser diffraction, and dynamic light scattering, or a similar technique. The actual behavior (e.g., settling rate) of inclusion body preparations in an aqueous system under high pressure has not been previously characterized, and the relevance to scale effects and yield loss were not known. For example, the contributions of inclusion body size and flocculation state render the apparent size under high pressure ambiguous, as high pressure treatment might potentially accelerate inclusion body flocculation, or alternatively might quickly break up flocculants such that they have little potential to drive settling. Flocculation, which involves both recombinant protein-protein interactions and interactions with cellular debris, can vary depending on the protein, method and conditions for cell disruption, buffer conditions, and processing steps. Further, the flocculation state of inclusion bodies traditionally has gone uncharacterized because it is largely irrelevant where inclusion bodies are solubilized with chaotropic agents or denaturing detergents. When flocculation is taken into account, inclusion bodies can have enormous size variability in the range of about 1μm to over 50 μm. Depending on the behavior of the flocculant under high pressure, the particular flocculant state of the inclusion bodies, and variability of the flocculation from batch-to-batch, there is a potential for large yield loss and loss of high pressure refold consistency, especially at large volumes.

The relevant size and/or physical properties of inclusion bodies can be difficult to characterize, in part because of the tendency to flocculate. For example, the size of inclusion bodies has been characterized as around 1 μm [Peternel and Komel, Isolation of biologically active nanomaterial (inclusion bodies) from bacterial cells. Microbial Cell Factories 2010 9:66; Balduino et al., Refolding by High Pressure of a Toxin Containing Seven Disulfide Bonds: Bothropstoxin-1 from Bothrops jararacussu Mol. Biotechnol. 48:228-234 (2011)], which would suggest a settling rate in non-denaturing conditions that would be suitable for high pressure refolding. The impact of flocculation, which can be responsible for much bigger particle sizes of up to 50 μm, on the settling rate during high pressure refolding has not been characterized. However, as shown herein, the size of inclusion bodies is sufficient to substantially impact the yield of pressure refolding, and that a stable dispersion of inclusion body preparations has significant positive affect on the refolding yield, especially at higher refolding volumes, and even under non-denaturing conditions.

Since previous reports of high pressure refold were conducted at small scale, the apparent protein concentration was not critical for refolding yields, relative to larger scales. There are reports where inclusion bodies were refolded with high pressure, each time without characterization of particle size or without attempts to control particle size, and without reference to flocculation or its effects on the process. One study attempted to prevent settling during high pressure UV spectroscopy through the use of hydroxy-ethyl-starch, which resulted in lower refolding yields due to preferential exclusion (St. John, Carpenter et al. 2001; Seefeldt, Crouch et al. 2007). When large-scale refolding of INF-beta-lb inclusion bodies was attempted, inclusion body settling was so significant that the refold process from inclusion bodies was ineffective, with yields of less than 5%. The problem was circumvented by adopting chemical-based solubilization techniques prior to pressure treatment. See U.S. Patent 8,273,561. The present inventors attempted other manners to circumvent inclusion body settling during high pressure treatment, including the development of equipment to agitate or mix under pressure, but these efforts proved to be difficult to scale. See for example, U.S. Patent 7,767,795 and US 2009/0215998.

The present invention prepares a stable inclusion body dispersion to avoid settling of inclusion body particles during high pressure treatment. Inclusion bodies often will readily flocculate (even under high pressure in some instances) with zeta potentials of less than -10 mV, and generally behave differently than other types of protein aggregates such as process-induced aggregates or protein precipitants. For example, while sub-visible particulates are often in the 0.2-10 μm range, these particles do not demand size control process steps due, for example, to their extremely low prevalence (typically <2%), which is too low to create any significant protein concentration gradient. Further still, non-inclusion body protein aggregates are generally cleaner material, are bound more loosely, have lower flocculating potential, and can be broken up with low shear forces, such that there is no need for harsh processes that have the potential of creating process-induced aggregation.

In certain embodiments, the invention involves creating a stable dispersion of an inclusion body preparation having substantial flocculation. For example, prior to dispersion, the inclusion body preparation may have a broad range of particle sizes of from less than about 1 μm to more than about 20 μm, or more than about 30 μm, 40 μm, 50 μm, or 100 μm. In some embodiments, greater than about 10%, greater than about 25%, or greater than about 50% of particles are greater than about 10 μm or greater than about 5 μm, and thus can significantly affect high pressure refolding yield.

Devices such as a Coulter Counter, imaging, laser diffraction, and dynamic light scattering can be used to characterize the particle size of the inclusion body dispersions, to ensure that particle sizes are in the appropriate size range, and maintain consistency from batch-to-batch. A Coulter Counter device, for example, provides an accurate measure of inclusion body size for high pressure refolding. A Coulter Counter measures particle diameter and distribution by measuring disturbances in electrical conductivity as particles move through a fixed cross-section. The gravitational force is a well defined constant. The solution viscosity and density of the particle and the fluid (water) is a function of solute composition. After conducting these calculations, an understanding of the settling velocities of all particles in the appropriate refolding buffer can be obtained.

The present invention in some embodiments incorporates a step of mechanical shear of inclusion bodies to prepare the inclusion body dispersion, for example through the use of a high pressure homogenizer. The inclusion body size and/or diameter and flocculation state after dispersion is such that the settling rate is less than about 1 cm per hour. In this manner, inclusion bodies that are loaded into high pressure refolds and pressure treated will have ample time to become solubilized prior to settling to the bottom of the pressure vessel. As shown herein, high pressure homogenization is an effective tool for improving high pressure refolding yield, including at larger scales. While high pressure processing in the absence of refolding solution conditions has been shown to form pressure-induced aggregates, which were further shown to be dense structures not amendable to high pressure refolding (Seefeldt, Crouch et al. 2007), the present disclosure shows that inclusion bodies treated by high pressure homogenization do not generally exhibit such properties.

Surprisingly, by creating an inclusion body dispersion prior to high pressure refolding, substantially improved yields can be obtained. In various embodiments, refolding yields of greater than about 30%, greater than about 40%, greater than about 50%, greater than about 60%, greater than about 70%, greater than about 80%, greater than about 90% or approaching 100% can be obtained. Such yields may be obtained even under non-denaturing conditions, and even at large scale. Such a result was not previously possible. In some embodiments, the volume of the pressure vessel used for high pressure refolding is about 5L or greater. In some embodiments, the volume of the pressure vessel used for high pressure refolding is about 10L or greater. In some embodiments, the volume of the pressure vessel used for high pressure refolding is about 15L or greater, or at least 25L or greater, or at least 50L or greater, or at least 100L or greater, or at least 125L or greater, or at least 150L or greater, or at least 200L or greater.

Thus, in various embodiments, the inclusion body dispersion is non-denaturing, that is, the dispersion does not contain a denaturing amount of detergents (e.g., SDS) or chaotropic agents (e.g., urea or guanidium HCl). In some embodiments, denaturing agents are not present in the dispersion, rendering their downstream removal unnecessary. In some embodiments, non-denaturing levels of a detergent or other agent that is denaturing at higher concentrations may be employed. For example, the detergents or other agent in the refold buffering system is not sufficient to solubilize the inclusion bodies.

The present invention in some embodiments, incorporates selecting solution conditions including at least one of pH, ionic strength, detergents, preferential excluding compounds, and polymers which can limit flocculation and provide a stable dispersion during inclusion body storage prior to refolding. In some embodiments, the solution conditions selected also are effective for refolding the protein under high hydrostatic pressure. In some embodiments, the solution conditions can be selected during inclusion body harvesting preventing the need for rehomogenization techniques. While not wanting to be bound by theory, the solution conditions are selected to increase the zeta potential of the inclusion body particles to be outside the range of about ±10 mV, about ±20 mV, about ±30 mV, or about ±40 mV, to facilitate a stable colloidal dispersion (Duffy and Hill 2011).

The protein produced through inclusion bodies can be any protein, for example, any protein to be produced at a manufacturing scale. In various embodiments, the protein is an industrial enzyme or a therapeutic protein. The therapeutic protein is a protein for human or veterinary therapy. In some embodiments, the protein has a solubility limit of less than about 100mg/ml (e.g., in an aqueous solution at a pH or about 7), or less than about 50 mg/ml, or less than about 20 mg/ml, or less than about 10 mg/ml, or less than about 5 mg/ml. In these or other embodiments, the protein has one or more disulfide bonds.

The protein may be a recombinant protein therapeutic, such as an immunoglobulin (e.g, a monoclonal antibody, which may be chimeric or humanized), an antigen-binding domain or single chain antibody, an Fc-domain containing protein (e.g., ENBREL), or other therapeutic protein. Where the protein comprises an antibody or antibody domain, the antibody or domain may be of any human isotype, such as an IgG isotype. Exemplary therapeutic proteins include an interleukin or interferon (e.g., an interferon-alpha, interferon-beta, or interferon-gamma), protein or peptide hormone or growth factor (e.g., insulin, GLP, erythropoietin, GM-CSF, or human growth hormone), clotting factor (e.g., Factor VII, Factor VIII), or enzyme for replacement therapy (e.g., uricase, MYOZYME, phenylalanine hydroxylase, phenylalanine ammonia lyase). The therapeutic protein may comprise full length proteins, or functional portions thereof, and may contain modifications known in the art for enhancing activity and/or stability of the molecule.

The recombinant therapeutic protein may be a large protein of one or more than one subunit. For example, the protein may have a size greater than about 500 kDa, 400 kDa, 200 kDa, 100 kDa, 75 kDa, 50 kDa, 40 kDa, 30 kDa, 20 kDa, 10 kDa, 5 kDa, 2 kDa, or 0.25 kDa. In certain embodiments, the recombinant protein comprises a plurality of polypeptide chains, which may optionally be connected by one or more disulfide bonds.

Exemplary therapeutic proteins include interferon-alpha; interferon-alpha 2a (Roferon-A; Pegasys); interferon-beta Ib (Betaseron); interferon-beta Ia (Avonex); insulin (e.g., Humulin-R, Humalog); DNAase (Pulmozyme); Neupogen; Epogen; Procrit (Epotein Alpha); Aranesp (2nd Generation Procrit); Intron A (interferon-alpha 2b); Rituxan (Rituximab anti-CD20); IL-2 (Proleukin); IL-I ra (Kineret); BMP-7 (Osteogenin); TNF-alpha Ia (Beromun); HUMIRA (anti-TNF-alpha MAB); tPA (Tenecteplase); PDGF (Regranex); interferon-gamma Ib (Actimmune); uPA; GMCSF; Factor VII, Factor VIII; Remicade (infliximab); Enbrel (Etanercapt); Betaferon (interferon beta- Ia); Saizen (somatotropin); Erbitux (cetuximab); Norditropin (somatropin); Nutropin (somatropin); Genotropin (somatropin); Humatrope (somatropin); Rebif (interferon beta Ia); Herceptin (trastuzumab); abatacept (Orencia) and Humira (adalimumab); Xolair (omalizumab); Avastin (bevacizumab); Neulasta (pegfilgrastin); Cerezyme (Imiglucerase); and motavizumab. The amino acid sequence and/or structure of such therapeutic proteins are known in the art.

The protein preparation is recovered from host cells in an insoluble form (e.g., inclusion bodies), and in some embodiments at a manufacturing scale. Expression of protein in inclusion bodies is well known. The host cells are microbial cells that express recombinant protein as inclusion bodies, such as *E coli*. High pressure homogenizers are commercially marketed to carry out cell lysis to recover inclusion bodies. For example, the host cell can be disrupted by mechanical means such as a Manton-Gaulin homogenizer or French press. It is preferred that the disruption process be conducted so that cellular debris from the host organism is so disrupted that it fails to sediment from the homogenate solution under low speed centrifugation sufficient to sediment the inclusion bodies. The inclusion bodies may be resuspended, washed and centrifuged again. The supernatant is discarded yielding a substantially pure preparation of inclusion bodies. For example, when using a high pressure homogenizer for cell lysis, the whole cells can be suspended in, for example, a 20 mM Tris, 2 mM EDTA buffer prior to processing. In other embodiments, chemical methods and/or sonication are used to disrupt the cells and recover the inclusion bodies. Cell disruption conditions can effect the properties of inclusion bodies [see Van Hee et al., Relation between cell disruption conditions, cell debris particle size, and inclusion body release, Biotechnol. Bioeng. 5;88(1): 100-10 (204)], and thus cell disruption is one factor to be optimized with respect to particular protein of interest to be refolded in accordance with the invention. The particle size of inclusion bodies has not traditionally been a process issue for recombinant protein production, because processes always include steps of inclusion body solubilization.

The inclusion body dispersion may be prepared by any suitable technique, as adjusted for the particular protein, and as needed to produce the requisite properties as described herein. In various embodiments, the dispersion technique operates by producing mechanical shear, such as a high speed blender, high pressure homogenizer, french press, colloid mill, high sheer disperser, or membrane homogenizer. Multiple passes through the equipment could be required to obtain the proper dispersion. The inclusion body dispersion is not substantially aided by denaturants or solubilizing compounds, and the inclusion bodies need not be solubilized and precipitated prior to high pressure treatment.

The process conditions for any particular homogenization technique or equipment should be established by evaluating particle size as disclosed herein. In various embodiments, the inclusion body particles in the dispersion are predicted to have a settling rate of 10 cm per hour or less (based on the average size of the particles, which can be determined as described herein), reducing the impact of settling on the refolding yield by high pressure treatment. In some embodiments, the inclusion body particles have a settling rate of 5 cm per hour or less, or a settling rate of 1 cm per hour or less. Thus, the majority of the inclusion body particles by mass have a size of 10 $\mu$m or less, or in other embodiments the majority of the inclusion body particles by mass have a size of 5 $\mu$m or less, or in other embodiments the majority of the inclusion body particles by mass have a size of 3 $\mu$m or less, or a size of 2 $\mu$m or less. In some embodiments, the particles of the dispersion consist essentially of particles of less than about 2.2 $\mu$m in size, which support colloidal properties of the dispersion, as well as particles in the range of about 2.2 $\mu$m to about 5 $\mu$m in size, which will not substantially settle during a high pressure refold reaction. In some embodiments, the majority of particles are less than about 2.2 $\mu$m. In some embodiments, at least about 30%, at least about 40%, at least about 50%, at least about 60%, at least about 70%, or at least about 80%, or at least about 90% of the particles are below the size of about 2.2 $\mu$m. Generally, the size of the inclusion body particles is substantially homogeneous, and does not substantially vary (e.g., with only about 5%, about 10%, or about 20% outliers) by more than about 5 $\mu$m, about 4 $\mu$m, about 3$\mu$m, or about 2$\mu$m.

The inclusion body dispersion can be stabilized either after harvest or after dispersion of a flocculated inclusion body preparation and their settling rate can be adjusted by addition of chemicals that modify the zeta potential such as pH, ionic strength, non-denaturing detergents, or viscosity-increasing agents. Many viscosity increasing reagents are also preferentially excluding compounds which stabilize aggregate structures and modulate protein volumes, impeding inclusion body solubilization and refolding (Arakawa and Timasheff 1982; Timasheff 1992; Timasheff 1993; Qoronfleh, Hesterberg et al. 2007; Seefeldt, Crouch et al. 2007). However, the invention in some aspects and embodiments involves achieving the balance between stabilizing the dispersion (e.g., avoiding settling and/or flocculation) without substantially stabilizing the inclusion body particles from disaggregation and refolding under high pressure. Viscosity-increasing agents include, for example, glycerol, sucrose, trehalose, poly-ethylene-glycol, which also disrupt the structure of water. In some embodiments, such agents are added in particular where a freeze-thaw step is included before high pressure treatment, to prevent flocculation. Chemicals that modify the zeta potential are well known in the art and work on the basis of increasing charge-charge repulsion. Zeta potentials of about ±20, about ±30, or about ±40 are sufficient to stabilize colloidal dispersions in various embodiments, and can be modified by pH, ionic strength, and charged refolding agents. Zeta potential can be quantified by light scattering techniques.

In some embodiments, the dispersion is stable for a period of at least one week, at least two weeks, at least one month, or at least two months, without further redispersion prior to high pressure refolding.

Because the inclusion body particles in the dispersion will not substantially settle, the apparent concentration of protein is maintained at below the protein solubility limit during high pressure treatment. For example, in various embodiments, the apparent concentration of protein throughout the high pressure vessel is less than about 50 mg/mL during high pressure treatment. In other embodiments, the apparent concentration of protein throughout the pressure vessel is less than about 30 mg/mL, or is less than about 10 mg/mL or less, or is less than about 5 mg/mL or less.

In some embodiments, the inclusion body dispersion is subjected to high pressure at from about 1000 bar to about 5,000 bar. In some embodiments, high pressure treatment of the inclusion body dispersion takes place without solubilization and/or precipitation of the inclusion bodies.

Generally, refolding takes place in a pressure window. In some embodiments the pressure window is from about 1000 bar to about 2500 bar, or about 1000 bar to about 2000 bar. In other embodiments, the window may be about 1250 bar to about 2250 bar, or about 1500 bar to about 2000 bar. Generally, the preparation is exposed to high pressure within a pressure window that results in disaggregation and refolding. The high pressure is generally below that which would irreversibly denature the protein. The high pressure conditions are selected to not induce aggregation, where the conditions include magnitude of high pressure, duration of high-pressure treatment, protein concentration, temperature, pH, ionic strength, chaotrope concentration (if used), surfactant concentration, buffer concentration, preferential excluding compounds concentration, or other solution parameters as described herein.

As used herein, the term "high pressure" means a pressure of at least about 250 bar. The high pressure treatment in accordance with embodiments of the invention may be at least about 250 bar of pressure, at least about 400 bar of pressure, at least about 500 bar of pressure, at least about 1 kbar of pressure, at least about 2 kbar of pressure, at least about 3 kbar of pressure, at least about 5 kbar of pressure, or at least about 10 kbar of pressure. "Atmospheric," "ambient," or "standard" pressure is defined as approximately 15 pounds per square inch (psi) or approximately 1 bar or approximately 100,000 Pascals. The selection of conditions for high pressure can be guided by techniques suitable for assessing refolding yield and active protein, including activity assays, ELISA's, SDS-PAGE, reverse-phase chromatography (RP), other HPLC based assays, size exclusion chromatography (SEC), reverse-phase chromatography (RP), other HPLC based assays, light scattering/obscuration, among others techniques.

High pressure vessels are commercially available (e.g. High Pressure Equipment Co., Erie, Pennsylvania). High-pressure techniques are described in U.S. Patent Nos. 6,489,450 and 7,064,192, U.S. Patent Application Publication No. 2004/0038333, and International Patent Application WO 02/062827. Certain devices have also been developed which are particularly suitable for refolding of proteins under high pressure; see International Patent Application Publication No. WO 2007/062174. Condition parameters to be adjusted for favorable high pressure treatment are described below. Horizontal vessels can be as available form National Calibration Inc. (Phoenix, AZ).

In some aspects and embodiments of the invention, the horizontal axis of the pressure vessel is at least twice the vertical axis. A pressure vessel with a large horizontal surface area, as shown herein, supports greater yield through high pressure folding. Vertical loading high pressure vessels have generally been used for conducting high pressure experiments and food pasteurization. These conventional vessels are easily filled these with both processing samples and pressure transmitting fluid. Fluid can be kept in the system while filling through the application of simple valves. There is also the phenomenon that hoop stress, and consequently the strength of a pressure vessel, is governed by the ratio of outer and inside diameter of the vessel and results in the volume of many vessels being controlled most easily by altering vessel height, not the vessel diameter. The combination of these properties results in the natural development of vertical pressure vessels that are "narrow" in dimension, maximizing height while minimizing diameter for a given volume.

As high pressure refolds become scaled, vertical loading pressure vessels become increasingly problematic as the apparent protein concentration increases with the increasing height of the pressure vessel. The problem is unique to high pressure refolding as high pressure pasteurization methods are independent of food location within the vessel. Many other high pressure reactions occur in solutions with constant density. The invention in some aspects proposes to implement horizontal loading vessels for large-scale refolding, decreasing the height of the pressure vessels, increasing the surface area on the bottom of the vessel, and thus decreasing the apparent protein concentration relative to vertically loaded vessels. To this point, pressure vessels have only been available that are of the vertical configuration. The advent of horizontal loading pressure vessels at large scale are particularly suited for high pressure refolding - not due to increased loading rates, the typical application, but rather the ability to decrease the apparent concentration in the refolding system.

The protein concentration of the inclusion body dispersion that is subjected to high pressure treatment may generally be in the range of about 0.1 mg/ml to about 50 mg/ml, such as at least about 1.0 mg/ml, at least about 5.0 mg/ml, at least about 10 mg/ml, or at least about 20 mg/ml, but will be dependent on the protein's solubility limit.

The duration of high pressure treatment may be selected for based on refold yield. Generally, high pressure treatment may be conducted for about 15 minutes to about 50 hours, or possibly longer. In some embodiments, the duration of high pressure treatment is up to about 1 week, about 5 days, about 4 days, about 3 days, etc.). Thus, in some embodiments, the duration sufficient for refolding is from about 2 to about 30 hours, from about 2 to about 24 hours, from about 2 to about 18 hours, or from about 1 to about 10 hours.

The solution components may be one or more agents selected from one or more stabilizing agents, one or more buffering agents, one or more surfactants, one or more disulfide shuffling agent pairs, one or more salts, or combinations of two or more of the foregoing. Where such component(s) are not pharmaceutically acceptable, the added component(s) should be removable from the protein preparation prior to administration as a pharmaceutical. Such components may be removed by dialysis or chromatography. In some embodiments, the inclusion body dispersion does not contain chaotropes or denaturing detergent. Exemplary agents include, but are not limited to, buffers (examples include, but are not limited to, phosphate buffer, borate buffer, carbonate buffer, citrate buffer, HEPES, MEPS), salts (examples include, but are not limited to, the chloride, sulfate, and carbonate salts of sodium, zinc, calcium, ammonium and potassium), chaotropes (when employed examples include, urea, guanidine hydrochloride, guanidine sulfate and sarcosine), and stabilizing agents (e.g., preferential excluding compounds, etc.). The solution components may also be selected to facilitate refolding and stabilize the inclusion body dispersion.

Amino acids can be used to prevent reaggregation and facilitate the dissociation of hydrogen bonds. Typical amino acids that can be used, without limitation, are arginine, lysine, proline, glycine, histidine, and glutamine or combinations of two or more of the foregoing. In some embodiments, the free amino acid(s) is present in a concentration of about 0.1 mM to about the solubility limit of the amino acid, and in some variations from about 0.1 mM to about 2 M. The optimal concentration is a function of the desired protein and should favor the native conformation. Amino acids have charge associated as a function of the solution and the pKa of the leaving groups and will impact zeta potential, and thus dispersion stability, by adjusting the ionic strength and dielectric of the solution.

Preferentially excluding compounds can be used to stabilize the native conformation of the protein of interest. Possible preferentially excluding compounds include, but are not limited to, sucrose, hexylene glycol, sugars (e.g., sucrose, trehalose, dextrose, mannose), and glycerol. The range of concentrations that can be use are from 0.1 mM to the maximum concentration at the solubility limit of the specific compound. Exemplary concentrations include those that are consistent with physiological osmolality. The optimum preferential excluding concentration is a function of the protein of interest. Many viscosity increasing reagents are also preferentially excluding compounds which stabilize aggregate structures and modulate protein volumes, impeding inclusion body solubilization and refolding (Arakawa and Timasheff 1982; Timasheff 1992; Timasheff 1993; Qoronfleh, Hesterberg et al. 2007; Seefeldt, Crouch et al. 2007). However, in some embodiments, the invention involves achieving the balance between preventing flocculation and settling by applying a concentration of one or more preferential excluding compounds, but avoiding concentrations of preferential excluding compounds that might otherwise stabilize the inclusion body particles making them more resistant to high pressure treatment.

Buffering agents may be present to maintain a desired pH value or pH range. Numerous suitable buffering agents are known to the skilled artisan and should be selected based on the pH that favors (or at least does not disfavor) the native (monomeric) conformation of the protein of interest. Either inorganic or organic buffering agents may be used. Thus, in some embodiments, at least one inorganic buffering agent is used (e.g., phosphate, carbonate, etc.). In certain embodiments, at least one organic buffering agent is used (e.g., citrate, acetate, Tris, MOPS, MES, HEPES, etc.). Additional organic and inorganic buffering agents are well known to the art. Buffering components adjust the pH of the solution and controls the net charge and the charge distribution on inclusion body particulates. As buffer components are also charged, they also modify the solution ionic strength. Both factors influence the zeta potential and should be tested to evaluate their impact on the stability of dispersion.

A surfactant, a surface active compound, may also be employed to reduce the surface tension of the water. Surfactants may also improve the solubility of the protein of interest. Surfactants may be used at concentrations above or below their critical micelle concentration (CMC), for example, from about 5% to about 20% above or below the CMC. However, these values will vary dependent upon the surfactant chosen, for example, surfactants such as, beta-octylgluco-pyranoside may be effective at lower concentrations than, for example, surfactants such as TWEEN-20 (polysorbate 20). The optimal concentration is a function of each surfactant, which has its own CMC. In some embodiments, surfactants are not employed. Where used, the potential surfactants include nonionic (including, but not limited to, t-octylphenoxypolyethoxy-ethanol and polyoxyethylene sorbitan), anionic (e.g., sodium dodecyl sulfate) and cationic (e.g., cetylpyridinium chloride) and amphoteric agents. Suitable surfactants include, but are not limited to deoxycholate, sodium octyl sulfate, sodium tetradecyl sulfate, polyoxyethylene ethers, sodium cholate, octylthioglucopyranoside, n-octylglucopyranoside, alkyltrimethylanmonium bromides, alkyltrimethyl ammonium chlorides, non-detergent sulfobetaines, and sodium bis (2 ethylhexyl) sulfosuccinate. In some embodiments the surfactant may be polysorbate 80, polysorbate 20, sarcosyl, Triton X-100, β-octyl-glucopyranoside, or Brij 35. Surfactants have also been shown to strongly impact the zeta potential and but must be tested independently as they modify both the hydrophobicity and charge of the protein. The zeta potential is also affected by the amount of anionic surfactant in the solution. See Malhotra and Coupland, The effect of surfactants on the solubility, zeta potential, and viscosity of soy protein isolates, Food Hydrocolloids 18 (2004) 101-108.

Where the desired protein contains disulfide bonds in the native conformation it is generally advantageous to include at least one disulfide shuffling agent pair in the mixture. The concentrations of thiol-reactive compounds are sufficiently low to not alter the zeta potential significantly.

The methods described herein can be performed at a range of temperature values, depending on the particular protein of interest. For example, the protein can be refolded (e.g., disaggregated) at various temperatures, including at about room temperature, about 25 °C, about 30 °C, about 37 °C, about 50 °C, about 75 °C, about 100 °C, or about 125 °C. Generally, the temperature will range from about 0 to about 50 °C, about 10 to about 37 °C, or about 20 to about 30 °C.

In some embodiments, the temperature can range from about 20 °C to about 100 °C without adversely affecting the protein of interest, provided that prior to return to room temperature, the mixture is brought to a temperature at which it will not freeze.

Although increased temperatures are often used to cause aggregation of proteins, when coupled with increased hydrostatic pressure increased temperatures can enhance refolding recoveries effected by high pressure treatment, provided that the temperatures are not so high as to cause irreversible denaturation. Generally, the increased temperature for refolding should be about 20 °C lower than the temperatures at which irreversible loss of activity occurs. Relatively high temperatures (for example, about 60°C to about 125 °C, may be used while the solution is under pressure, as long as the temperature is reduced to a suitably low temperature before depressurizing. Such a suitably low temperature is defined as one below which thermally-induced denaturation or aggregation occurs at atmospheric conditions. Increases in temperature increase the impact of Brownian motion of the solution and could increase the stability of some dispersions.

Where the reduction in pressure is performed in a continuous manner, the rate of pressure reduction can be constant or can be increased or decreased during the period in which the pressure is reduced. In some variations, the rate of pressure reduction is from about 5000 to 2000 bar/1 sec to about 5000 to 2000 bar/4 days (or about 3 days, about 2 days, about 1 day). In some embodiments, the pressure reduction may be approximately instantaneous, as in where pressure is released by simply opening the device in which the sample is contained and immediately releasing the pressure.

Where the reduction in pressure is performed in a stepwise manner, the process comprises dropping the pressure from the highest pressure used to at least a secondary level that is intermediate between the highest level and atmospheric pressure. The goal is to provide an incubation or hold period at or about this intermediate pressure zone that permits a protein to adopt a desired conformation.

In some embodiments, where there are at least two stepwise pressure reductions there may be a hold period at a constant pressure between intervening steps. The hold period may be from about 10 minutes to about 50 hours (or longer, depending on the nature of the protein of interest). In some embodiments, the hold period may be from about 2 to about 24 hours, from about 2 to about 18 hours, or from about 1 to about 10 hours.

In particular embodiments, constant pressure after the stepwise reduction is from about four-fifths of the pressure immediately prior to the stepwise pressure reduction to about one-tenth of prior to the stepwise pressure reduction. For example, constant pressure is at a pressure of from about four-fifths to about one-fifth, from about two-thirds to about one-tenth, from about two-thirds to about one-fifth of the pressure immediately prior to the stepwise pressure reduction. Where there is more than one stepwise pressure reduction step, the pressure referred to is the pressure immediately before the last pressure reduction (e.g., where 2000 bar is reduced to 1000 bar is reduced to 500 bar, the pressure of 500 bar is one-half of the pressure immediately preceding the previous reduction (1000 bar)).

Where the pressure is reduced in a stepwise manner, the rate of pressure reduction (e.g., the period of pressure reduction prior to and after the hold period) may be in the same range as that rate of pressure reduction described for continuous reduction (e.g., in a non-stepwise manner). In essence, stepwise pressure reduction is the reduction of pressure in a continuous manner to an intermediate constant pressure, followed by a hold period and then a further reduction of pressure in a continuous manner. The periods of continuous pressure reduction prior to and after each hold period may be the same continuous rate for each period of continuous pressure reduction or each period may have a different reduction rate. In some embodiments, there are two periods of continuous pressure reduction and a hold period.

In certain embodiments, each continuous pressure reduction period has the same rate of pressure reduction. In other embodiments, each period has a different rate of pressure reduction. In particular embodiments, the hold period is from about 8 to about 24 hours. In some embodiments, the hold period is from about 12 to about 18 hours.

The refolding yield in accordance with the various embodiments of the invention may be greater than 50%, greater than 60%, greater than 75%, greater than 80%, or greater than 90%, or approaching 100% in some embodiments. Refolding yields may be determined by various techniques, including characterization of aggregates in solution. Techniques include activity assays, ELISA's, SDS-PAGE, reverse-phase chromatography (RP), other HPLC based assays, size exclusion chromatography (SEC), reverse-phase chromatography (RP), other HPLC based assays, light scattering/obscuration, among others techniques.

The protein preparation may be evaluated by size-exclusion chromatography and gel permeation chromatography, which can estimate molecular weights and aggregation numbers of proteins. Such techniques also separate out various protein aggregates. See Wu, C-S. (editor), Handbook of Size Exclusion Chromatography and Related Techniques, Second Edition (Chromatographic Science), Marcel Dekker: New York, 2004 (particularly chapter 15 at pages 439-462 by Baker et al., "Size Exclusion Chromatography of Proteins") and Wu, C-S. (editor), Column Handbook for Size Exclusion Chromatography, San Diego: Academic Press, 1999 (particularly Chapters 2 and 18).

Light obscuration can also be used to measure protein aggregation of the preparation; see Seefeldt et al., Protein Sci. 14:2258 (2005); Kim et al., J. Biol. Chem. 276: 1626 (2001); and Kim et al., J. Biol. Chem. 277: 27240 (2002).

Many methods of gel electrophoresis (e.g., denaturing or non-denaturing PAGE) can be employed to analyze proteins and protein aggregation. Native PAGE (non-denaturing PAGE) can be used to study non-covalently linked aggregates. See, e.g., Hermeling et al. J. Phar. Sci. 95:1084-1096 (2006); Kilic et al., Protein Sci. 12:1663 (2003); Westermeier, R., Electrophoresis in Practice: A Guide to Methods and Applications of DNA and Protein Separations 4th edition, New York: John Wiley & Sons, 2005; and Hames, B.D. (Ed.), Gel Electrophoresis of Proteins: A Practical Approach, 3rd edition, New York: Oxford University Press, USA, 1998.

In some embodiments, the invention is employed in conjunction with protease cleavage of recombinant fusion proteins in inclusion bodies, as described in U.S. Patent 7,829,681. In some embodiments, the invention is used in conjunction with the Npro autoprotease technology (Boehringer Ingelheim). For example, the inclusion body protein may be expressed as a fusion protein having a protease cleavage site, and the inclusion body protein subjected to high pressure together with a protease sufficient for protease cleavage to liberate the protein of interest. The fusion protein may comprise the protein of interest (e.g., the therapeutic protein or industrial enzyme) and the protease. In some embodiments, the protease is pestivirus protease.

In these or other embodiments, the fusion protein may contain or more of a HIS-tag, maltose-binding protein, thioredoxin, glutathione-s-transferase, DsbA, gphD, FLAG, calmodulin binding protein, streptag II, pestivirus protease, HA-tag, Softag1, Softag 3, c-myc, T7-tag, S-tag, NusA, chitin-binding domain, xylanase 10A, tobacco etch virus, and ubiquitin.

EXAMPLES

*Example 1: Inclusion Body Preparation*

Four inclusion body (IB) samples were initially produced using known techniques as a basis to compare the effect on particle size, setting rate, and yield upon subsequent high pressure refolding using (1) untreated IB protein preparations as a negative control; and (2) treated IB protein preparations as stable protein preparations or dispersions according to the present invention using additional steps of high shear mechanical processing, in this case, as a non limiting example, using additional high shear homogenization using a NIRO Panda treatment, as presented below. Unexpectedly, the additional treatment of IB protein preparations using additional high shear mechanical processing resulted in lower or decreased particle size, settling rate, and/or apparent protein concentration during high pressure refolding, that provided a significant increase in refolded protein yields.

In these experiments the four IB protein preparations included: (i) granulyte colony stimulation factor (rhG-CSF), (ii) Fab 1664 (a proprietary Fab construct), (iii) Inclusion body A, and (iii) Inclusion body B. Expression of inclusion bodies for each IB protein preparation in the cytoplasm of E. coli was conducted using standard laboratory procedures. IB protein preparation for Fab 1664 and rhG-CSF was conducted using homogenization. After homogenization, inclusion bodies were purified via centrifugation at 10,000 x g by washing and resuspending in wash buffer containing 50mM Tris, pH 8.0, ImM EDTA 3 times and the inclusion bodies were stored at -20°C prior to use. Washing protocols for the production of Fab 1664 and rhG-CSF were also consistent with known methods. The frozen cell paste for each protein was resuspended in lysis buffer (50 mM Tris buffer, at pH 8.0, 1 mM EDTA and 1 mM PMSF) at a pellet:buffer ratio of 1:10 (w/v). The inclusion bodies containing rhG-CSF or rhG-CSF were recovered from the cells using a Microfluidizer M-110P at a pressure of 14,000 psi. Prior to centrifugation, a 1:2 dilution of the lysate was carried out to reduce viscosity and to obtain a better yield of inclusion bodies. The resulting lysate solution was centrifuged at 48,000 × g for 30 minutes at 4°C to pellet the inclusion bodies using a Thermo Sorvall RC6+ Centrifuge. The supernatant was discarded, and the fraction containing the inclusion bodies was subjected to a three-step wash procedure to eliminate endotoxins, proteins and DNA of the host cells. In all steps, the pellet was suspended in a series of washing buffers at 1:40 (w/v) ratio at room temperature, stirred for 30 min and re-pellet by centrifugation. The first buffer was comprised of: 50 mM Tris, pH 8.0, 5 mM EDTA and 2% Triton ×-100. The composition of the second buffer was 50 mM Tris, pH 8.0, 5 mM EDTA, 1% sodium deoxycholate. In the third wash step, the wash buffer contained 50 mM Tris buffer, pH 8.0, 5 mM EDTA, and 1 M NaCl. After centrifugation, the inclusion bodies were stored at 4°C until resuspension in water at a concentration of 15 mg/ml. Inclusion body A and B protein preparations were also generated in methods consistent with the protocols described here and/or known methods for generating IBs.

*Example 2: Inclusion Body Size Characterization*

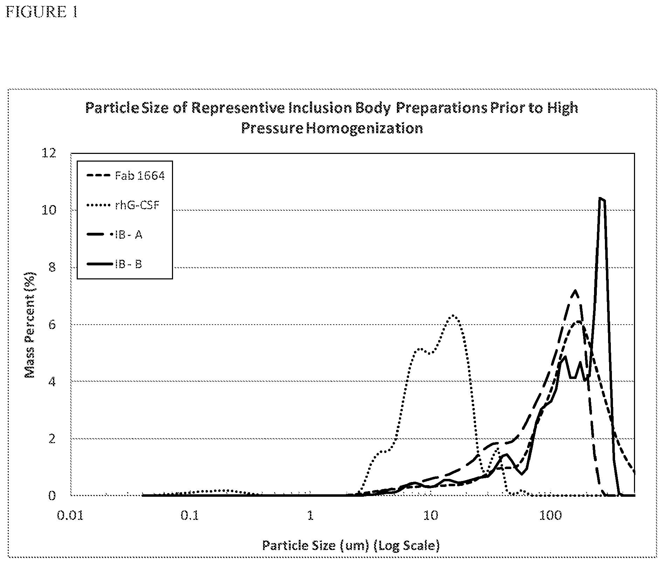

Inclusion body particle size for each of the four IB protein preparations was measured using the LS230 Coulter Particle Counter manufactured by Beckman. The instrument was washed with 0.22μm filtered water and background subtracted. Inclusion body suspensions were vortexed and particles were transferred to the detector using a transfer pipet until the particles counts were between 35-60% PID at a pump velocity ranging from 50-70% to prevent settling. For the analysis no sonication was conducted on the sample chamber. The model used for calculating particle size distributions used a solution refractive index of 1.33 (for water) and a sample refractive index of 1.5 (for protein). For each run, three ninety second averaged particle size distributions were taken for each of the four IB protein preparations to quantify the particle distribution between 0.4-2000 μm with the mean obtained across the three samples and presented as a 95% confidence interval. Size distribution was presented as a volume percentage. Assuming constant density, the volume percentage can also be interpreted as a mass percentage. The size distribution for the four inclusion body preparations is shown in Figure 1. Mean particle sizes (based on mass percentage) for the inclusion body preparations was 133 +/-14, 13.4 +/- 2.4, 108 +/- 3, and 171 +/- 9 μm for inclusion bodies Fab 1664, rhG-CSF, Inclusion Body A and Inclusion Body B preparations respectively.

Figure 1 shows particle size distributions of four inclusion body preparations. All samples exhibit rapid settling as observed by visual inspection and as predicted by Stokes flow.

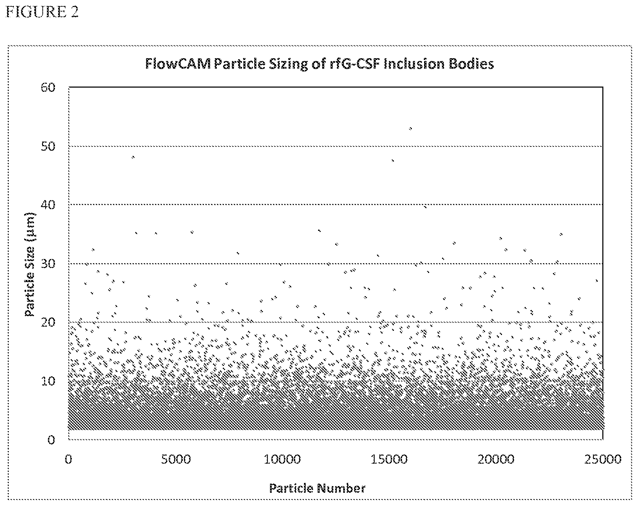

To confirm the Coulter Counter results, particle counting and sizing was conducted on rhG-CSF inclusion bodies using a FlowCAM (Fluid Imaging Technologies) instrument. The instrument flows the inclusion body suspension and takes microscopic pictures of the solution, determining particle size by optical measurement. Analysis was conducted by analyzing 200 μL of diluted solution flowed through a 300 micron flow cell at 0.15 mL/min. FlowCAM dark setting was 15 and light setting was 17. The minimum particle size detected was 2 microns, and the maximum detected was 10,000 microns. The resulting size distribution is shown in Figure 2. The FlowCAM analysis demonstrates that particle sizes range between 5-50 μm for rhG-CSF inclusion, matching the Coulter Counter results.

*Example 3: Settling Rates of Inclusion Body Preparations*

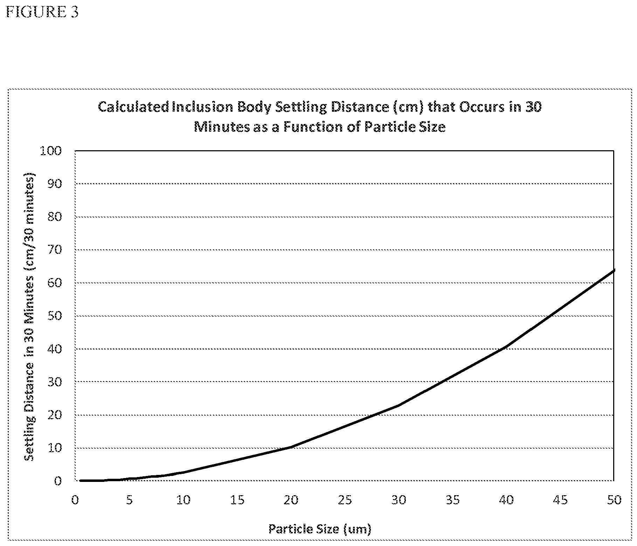

The settling of a Stokes particle in a non-moving fluid is completely characterized by the Stokes equation (de Nevers 1970): where V is the velocity of the particle, D is the diameter of the particle, g the gravitation constant, $\rho_{part}$ the density of the particle, and $\rho_{fluid}$ the density of the fluid. Studies by Middleburg demonstrated that density of an inclusion body is 1.26 g/ml (Thomas, Middelberg et al. 1990). Using 1 g/ml as the density of water and assuming a settling time of thirty minutes, the distance travelled by a particle as a function of its diameter is shown in Figure 3. The Stokes equation demonstrates that particles of 5 μm in diameter settle 0.6 cm over a thirty minute period, a sufficient distance to generate a protein concentration gradient during a refold reaction. The settling distances for Fab 1664, rhG-CSF, Inclusion Body A and Inclusion Body B sample protein preparations without additional treatment according to the present invention was determined to be 429, 4.5, 307, and 734 cm respectively, sufficient to result in inclusion bodies to form a boundary layer at the bottom of a non-mixed high pressure refold reaction after 30 minutes, which would result in reduced yields of refolded protein. All samples were observed to settle rapidly via visual inspection.

*Example 4: Preparation of Stable Protein Preparations or Dispersions of Inclusion Body Preparations According to the Present Invention*

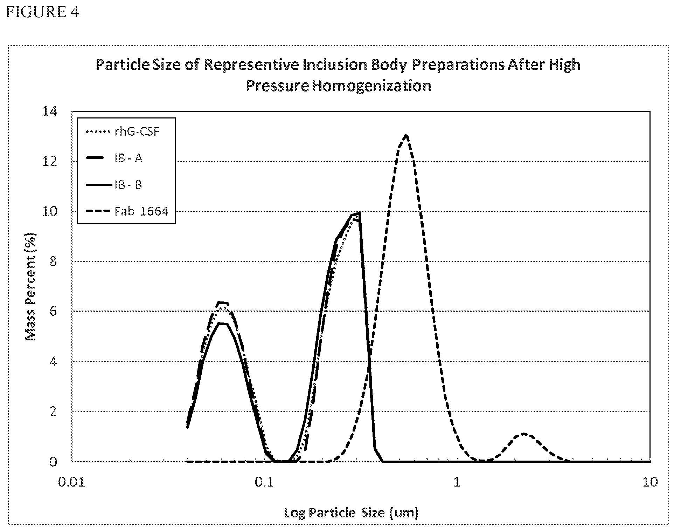

The inclusion body protein preparations shown in Example 2 (Fab 1664, rhG-CSF, Inclusion Body B, and Inclusion Body A) were reprocessed according to the present invention by additional high shear mechanical processing as high shear homogenization, as a non-limiting example, using a NIRO Panda Processor (1st stage only) at a backpressure of 1000 bar. Each IB protein preparation sample that had been treated according to the present invention to provide stable protein preparations or dispersions was collected and then stored at 25°C for no greater than five hours and retested (for showing unexpected and/or improved size distribution over non treated IB preparations) using the identical method as described in Example 3. The size distribution after high shear mechanical processing according to the invention is shown in Figure 4. Unexpectedly and in sharp contrast to non treated IB preparations, all inclusion body particles of the stable protein preparations or dispersions of the four proteins according to the present invention are less than 2 μm in size, with a majority of the particles less than 1 μm in size. Based on the Stokes equation and Eqn. 3, these particles would have settling rates of less than 1 μm after thirty minutes, preventing the formation of a protein boundary layer or concentration gradient during the course of a typical non-mixed high pressure refolding reaction, and also found to increase protein yields after refolding. All preparations remained homogenous after dispersion via visual inspection.

*Example 5: Effect of Particle Size Distribution on the High Pressure Refolding of Fab 1664 According to the Present Invention*

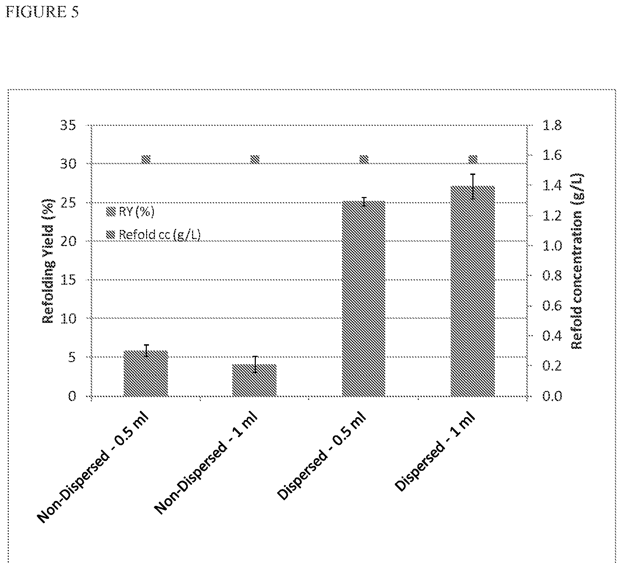

Fab 1664 Inclusion Bodies (before (non-treated designated "non-homogenized") and after (treated as per the invention designated as "homogenized") high shear mechanical processing of the invention) were quantified for protein concentration by densitometry using SDS-PAGE with purified Fab 1664 as a reference standard. Quantification was conducted to ensure that the dispersion process did not alter the protein concentration. Refold suspensions of Fab 1664 heavy and light chain (approximate 1:1 mass ratio) were made at a constant protein concentration of 1.6 g/ in buffer containing 50 mM CHES (pH 9.0), 4mM cysteine, 350mM arginine and pressure treated at 3000 bar for thirty minutes to facilitate aggregate dissociation, depressurized to 1500 bar for four hours to facilitate chain assembly, and incubated overnight at atmospheric pressure to facilitate disulfide formation. Refolding volumes of 0.5 and 1ml were chosen for the agglomerated and high shear mechanical processed treated ("homogenized") inclusion bodies in sample containers with an inner diameter of 1.45 mm to demonstrate the dependence of scale on the refold yield. The 0.5ml refold had a height of 2.8 cm while the 1ml refold had a height of 5.6 cm and all four samples were pressure treated in a vertical position. For agglomerated samples, the 0.5ml and 1ml refold resulted in 0.8 and 1.6 mgs of protein forming a thin layer at the bottom of the sample container respectively. After high pressure refolding, Fab 1664 refolding yields were determined using cation exchange chromatography (CEX). CEX was performed on an Agilent 1100 HPLC with 280nm detection. Separation was conducted using a Dionex ProPac WCX-10 4X250 mm column with a gradient increasing by 1% B/min where buffer A contains 50mM MES (pH 6.3) and buffer B contains 50mM MES (pH 6.3) and 250mM NaCl. Refolding yields as a function of Fab 1664 inclusion body particle size and refold volume are shown in Figure 5. Stable protein preparation or dispersion prior to refolding according to the invention is unexpectedly found to increase refolding yields significantly in a manner that is scale independent.

In particular, the treatment of IB protein preparations according to the present invention using high shear mechanical processing to provide stable protein preparations or dispersions ("non-homogenized"), prior to high pressure refolding, is unexpectedly found to improve the high pressure refolding yield of Fab 1664 from 5% to 25%, independent of scale in some embodiments. In contrast, non-treated IB preparations ("non-homogenized") of Fab 1664 inclusions unexpectedly settle too quickly, decreasing the apparent protein concentration to be well over 1.6 g/L and results in refolding yields of approximately 5%. When the refolding volume is increased to 1 ml, increasing the refold container height to 5.6 cm and resulting in 1.6 mgs of protein in the boundary layer, the refolding yield decreases further.

*Example 6 - Effect of Particle Size Distribution on the High Pressure Refolding of rhG-CSF According to the Present Invention*

rhG-CSF (before and after high pressure homogenization) were quantified for protein concentration by densitometry using SDS-PAGE with purified rhG-CSF as a reference standard. Quantification was conducted to ensure that the high shear mechanical processing of the IB preparation according to the present invention did not alter the protein concentration. Refold suspensions were made at a constant protein concentration of 0.45 mg/ml in non-optimized high pressure refolding conditions of 50mM CHES (pH 10.0), 1M urea, 0.01% Triton X-100, and 4mM cysteine. Treated ("homogenized") rhG-CSF stable protein preparations or dispersions according to the invention remained suspended after sample preparation whereas the non-treated control ("non-homogenized") preparations settled to the bottom of the container. The samples were pressure treated at 2500 bar for sixteen hours at volumes of 200μL, 600μL, 1000μL, and 10ml.

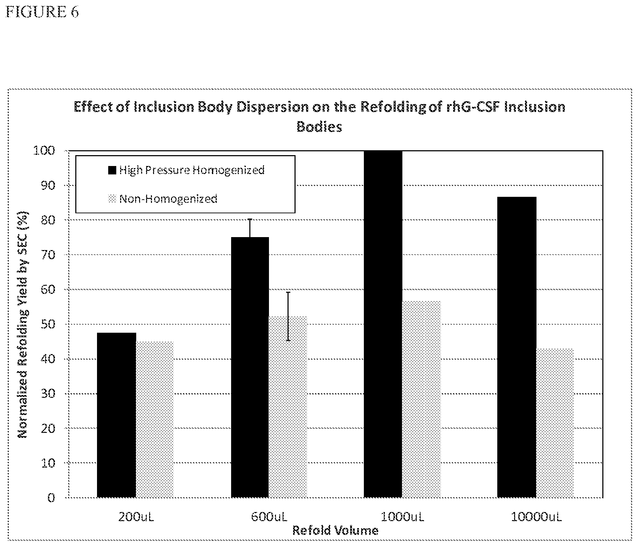

After refolding, yield was assessed using size exclusion chromatography (SEC-HPLC), reverse-phase chromatography (RP-HPLC), and densitometry using SDS-PAGE, with rhG-CSF as a reference standard. On average, high pressure refolding of the treated ("homogenized") stable protein preparations or dispersions of the invention resulted in yields of 22% and use of the control non-treated ("non-homogenized") IB preparations decreased yields to 7% by SDS-PAGE. SEC analysis was conducted using 20mM phosphate (pH 6.8), 150mM NaCl, and 0.01% Tween 20 as a mobile phase with a Tosohaas 3000 SWXL column with rhG-CSF as a reference standard. Normalized refolding yields as a function of stable protein preparation or dispersion and refolding volume are shown in Figure 6. Stable protein preparation or dispersion of rhG-CSF inclusion bodies increases refolding yields and minimizes scale impacts.

It is expected that the lower refolding yields observed at the 200μL volume are the result of air bubbles solubilized during high pressure treatment and altering the redox chemistry. For samples with refolding volumes between 600 and 10,000μL, two conclusion can be drawn. First, dispersion of rhG-CSF inclusion bodies increases the refolding yield for all volumes. Second, the refolding yield is maintained from 600 to 10000μL for the treated ("homogenized") samples made according to the invention, whereas there is a decrease in yield as the volume is increased in the case of the non-treated control ("non-homogenized") inclusion body preparations. It is believed that the decreased yield is the result of the formation of a protein boundary layer and an increase in the apparent protein concentration.

*Example 7: Use of Glycerol to Prevent Flocculation During Freeze/Thaw*

Studies by Middelberg and others demonstrate that cell homogenate, which includes inclusion bodies, contains particles in the size range of 0.2-0.6$\mu$m (Thomas, Middelberg et al. 1990; Bowden, Paredes et al. 1991). During processing, including centrifugation, washing, and freeze-thaw, inclusion bodies can flocculate, resulting in large particle size distributions (Figure 1).

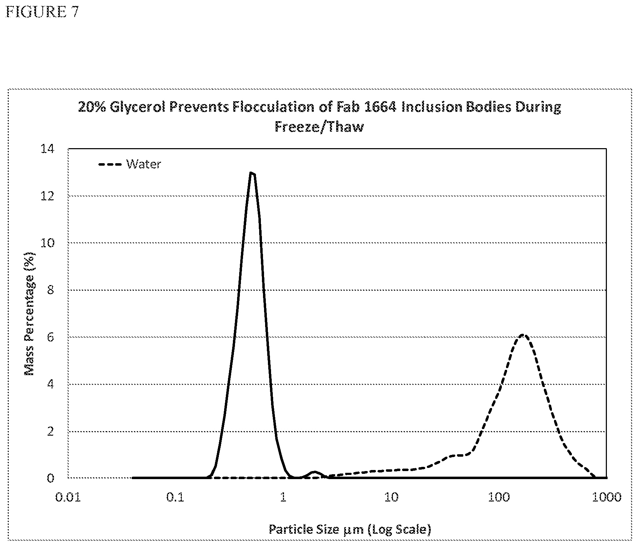

It is now found that flocculation of an IB protein preparation, e.g., Fab 1664, can be prevented if the IB protein preparation is frozen in a 15-30%, e.g., 20% glycerol solution prior to refolding (Figure 7). In contrast, Fab 1664 inclusion bodies frozen in water flocculated significantly. As an alternative to redispersion after flocculation, additives and solution chemistry not limited to at least one of pH, ionic strength, detergents, and dielectric and viscosity modifiers can be added to prevent flocculation during processing. Glycerol could have the added benefit that it disrupts the structure of water during freezing/thaw cycles.

References

Arakawa, T. and S. N. Timasheff (1982). "Stabilization Of Protein-Structure By Sugars." Biochemistry 21(25): 6536-6544.

Bowden, G. A., A. M. Paredes, et al. (1991). "Structure and morphology of protein inclusion-bodies in escherichia coli." Biotechnology 9(8): 725-730.

de Nevers, N. (1970). Fluid Mechanics for Chemical Engineers, McGraw Hill.

Duffy, D. and A. Hill (2011). Suspension Stability: Why Particle Size, Zeta Potential and Rheology are Important, www.malvern.comf.

Laidler, K. and J. Meiser, Eds. (1995). Physical Chemistry 2nd Edition. Boston, MA, Houghton Mifflin Company.

Qoronfleh, W., L. K. Hesterberg, et al. (2007). "Confronting high-throughput protein refolding using high pressure and solution screens." Protein Expression and Purification 55: 209-224.

Seefeldt, M. B., C. Crouch, et al. (2007). "Specific volume and adiabatic compressibility measurements of native and aggregated recombinant human interleukin-1 receptor antagonist: Density differences enable pressure-modulated refolding." Biotechnology And Bioengineering 98(2): 476-485.

Seefeldt, M. B., J. Ouyang, et al. (2004). "High-pressure refolding of bikunin: Efficacy and thermodynamics." Protein Science 13(10): 2639-2650.

St. John, R. J., J. F. Carpenter, et al. (2001). "High pressure refolding of recombinant human growth hormone from insoluble aggregates - Structural transformations, kinetic barriers, and energetics." Journal of Biological Chemistry 276(50): 46856-46863.

St. John, R. J., J. F. Carpenter, et al. (1999). "High pressure fosters protein refolding from aggregates at high concentrations." Proceedings of the National Academy of Sciences of the United States of America 96(23): 13029-13033.

Thomas, J. C., A. P. J. Middelberg, et al. (1990). "Sizing Biological Samples by Photosedimentation Techniques." Biotechnology Progress 6: 255-261.

Timasheff, S. N. (1992). "Water as Ligand - Preferential Binding and Exclusion of Denaturants in Protein Unfolding." Biochemistry 31(41): 9857-9864.

Timasheff, S. N. (1993). "The Control of Protein Stability and Association by Weak-Interactions with Water - How Do Solvents Affect These Processes." Annual Review of Biophysics and Biomolecular Structure 22: 67-97.

*Example 8: Effect of Different Stable Protein Preparation or Dispersion Approaches on Particle Size Distributions and Increased Yields After Protein Refolding According ot the Present Invention*

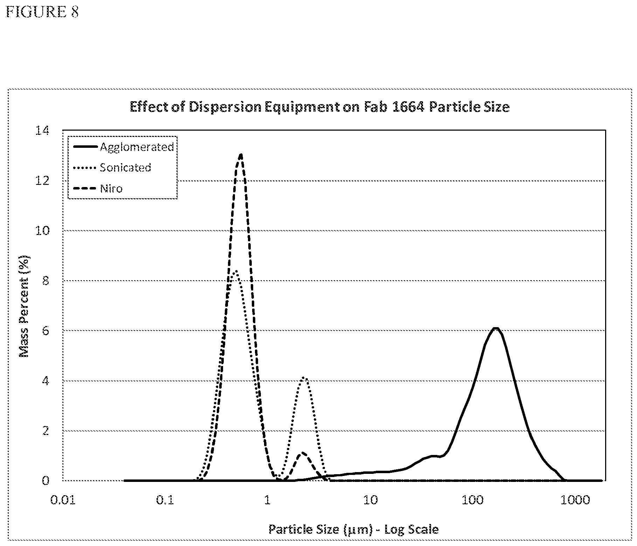

Agglomerated Fab 1664 inclusion bodies were processed using a probe sonicator (1ml scale) and a Niro Panda at 800 psi (50ml scale) to provide IB protein preparations. After processing, the inclusion bodies were assessed for particle size as shown in Figure 8. The processing approach used altered the size distribution, with sonication resulting in incomplete dispersion and a higher percentage of particles present in the 2-4 $\mu$m size range. The higher proportion of 2-4 $\mu$m sized particles is significant in a 500uL refold, as the total refold height is only 2.8 cm and 2 and 4 $\mu$m particles settle a distance of 0.1 and 0.4 cm respectively, resulting in an increase in the apparent protein concentration during the refold step. Refolding yields for agglomerated, sonicated, and Niro homogenized Fab 1664 refolding yields pressure treated with identical conditions as described in Example 5 resulted in yields of 5.9% +/- 0.7%, 13.8% +/- 0.2%, and 25.2% +/- 0.5%. The incomplete dispersion after sonication resulted in a larger proportion of particle sizes and decreased yields relative to a more monodisperse sample. Accordingly, it is unexpectedly discovered that sonication can reduce protein refolding yields and thus providing a method according to the present invention that provide high yields without sonication is preferably to methods that include sonication.

*Example 9: Experimental Design for Maximizing Dispersion Solution Stability*

Solution chemistry can be applied to either prevent flocculation of inclusion bodies during cell harvest, processing, or storage or after dispersion techniques have been employed to suspend the inclusion bodies. Protein preparations or dispersions can be stabilized by increasing the absolute zeta potential to prevent flocculation, or by increasing the solution viscosity to slow particle-particle collisions. Zeta potential is controlled by excluded volume, electrostatic interactions, van der Waals forces, entropic forces, and steric forces and values greater than +/- 30mV typically impart solution stability. As each protein has its independent pI, the charge repulsion for each protein must be optimized as a function of pH. Adjusting the pH of the solution away from the pI of the protein increases charge repulsion and colloidal stability. pHs of 4-10 should be tested, with care that long-term storage does not result in chemical modification or deamidation. The ionic strenth of the solution should be tested at low buffer concentration (10mM) and with the addition of 0.5 and 1M NaCl. The use of charged surfactants will increase the zeta potential dramatically, but are often denaturing (e.g SDS). Non-ionic surfactants can provide modest changes in zeta potential and should be tested in a stepwise manner above and below the CMC. The use of preferential excluding compounds and glycols will adjust the solution dielectric constant and thus the zeta potential and should be tested in the range of 0-1M in 0.25M increments. Experimentation can be conducted in a step-wise format or through the use of high throughput screening, fractional factorial and other statistical design. After inclusion bodies have been dispersed in the various solution conditions, zeta potential can be measured through dynamic light scattering to determine when the potential is maximized through either positive or negative charge. For each solution condition tested, the zeta potential should be tested, the particle distribution measured, and the samples placed on storage for one, two and four weeks at 25C, 4C, and through freeze-thaw cycles and then tested again for particle distribution using a Coulter Counter or other methods.

Viscosity modifying agents can also be used to stabilize flocculants by slowing protein-protein collisions, however care must be made to ensure that they do not modify the refold reaction as they are commonly preferentially excluding compounds. Viscosity modifying agents could be used effectively if they are diluted when mixing the inclusion bodies into the refold buffer. An non-exclusive list of viscosity modifiers to be tested include guar, xanthum gum, hydoxy-ethyl-startch, PEG, and agar.

**Claims**

1.   A method for protein production from inclusion bodies, comprising:

> a) providing a protein preparation comprising inclusion body particles,
> b) preparing an inclusion body dispersion by mechanical shear such that the inclusion body particles have a size of less than 10 $\mu$m, and
> c) exposing the inclusion body dispersion to high pressure in a pressure vessel, thereby disaggregating and refolding the inclusion body protein.

2.   The method of claim 1, further comprising selecting a refolding solution chemistry based on one or more of pH, ionic strength, and dielectric constant; wherein, optionally, the horizontal axis of the pressure vessel is at least twice the vertical axis.

3. The method of claim 1, wherein the inclusion body dispersion is non-denaturing, and wherein, optionally, the inclusion body dispersion does not contain chaotropes and/or detergent sufficient to solubilize the inclusion bodies in the absence of high pressure.

4. The method of any one of claims 1 to 3, wherein the volume of the pressure vessel is about 5 L or greater, about 10 L or greater, or about 50 L or greater.

5. The method of any one of claims 1 to 4, wherein the protein is a therapeutic protein that comprises an antigen binding region or an antibody Fc region.

6. The method of any one of claims 1 to 5, wherein the majority of the inclusion body particles in the dispersion have a settling rate of about 1 cm per hour or less.

7. The method of claim 6, wherein the majority of the inclusion body particles by mass have a size of 3 $\mu$m or less, or about 2.2 $\mu$m or less.

8. The method of claim 1, wherein the mechanical shear is generated by high pressure homogenization, microfluidizer processors, or fixed orifice or constant pressure processors.

9. The method of any one of claims 1 to 8, wherein the chemistry of the dispersion is adjusted by addition of one or more of non-denaturing detergent, buffering agent, salt, refolding co-agent, viscosity-increasing agent, or preferential excluding compound.

10. The method of claim 9, wherein the zeta potential of the particles is adjusted to be outside the range of $\pm$ 10 mV, or $\pm$ 20 mV, or $\pm$ 30 mV.

11. The method of any one of claims 9 to 10, wherein a preferential excluding compound is added at a concentration that prevents flocculation.

12. The method of claim 11, wherein the inclusion body dispersion is subjected to freeze/thaw and does not flocculate.

13. The method of any one of claims 1 to 12, wherein the inclusion body protein is a fusion protein having a protease cleavage site, and is subjected to high pressure together with a protease sufficient for protease cleavage.

14. The method of claim 13, wherein the protease is pestivirus protease.

15. The method of any one of claims 1 to 14, wherein the inclusion body particles are substantially homogeneous in size, and do not vary by more than 5 $\mu$m.

16. The method of any one of claims 1 to 15, wherein the dispersion is created without solubilizing or precipitating protein from inclusion bodies.

**Patentansprüche**

1. Ein Verfahren zur Proteinherstellung aus Einschlusskörpern, umfassend:

　　a) Bereitstellen eines Proteinpräparats, das Einschlusskörperpartikel umfasst,
　　b) Herstellen einer Einschlusskörperdispersion durch mechanische Scherung, so dass die Einschlusskörperpartikel eine Größe von weniger als 10 $\mu$m haben, und
　　c) Aussetzen der Einschlusskörperdispersion einem hohen Druck in einem Druckgefäß, wodurch das Einschlusskörperprotein disaggregiert und rückgefaltet wird.

2. Das Verfahren gemäß Anspruch 1 weiterhin umfassend das Auswählen einer Rückfaltungslösungschemie, die auf einem oder mehreren der Parameter pH, Ionenstärke und Dielektrizitätskonstante basiert, wobei die horizontale Achse des Druckgefäßes optional mindestens zweimal die vertikale Achse ist.

3. Das Verfahren gemäß Anspruch 1, wobei die Einschlusskörperdispersion nicht denaturierend ist, und wobei die Einschlusskörperdispersion optional keine Chaotropen und/oder Detergenz enthält, die ausreichen, um die Einschlusskörper in Abwesenheit von hohem Druck zu solubilisieren.

4. Das Verfahren gemäß einem der Ansprüche 1 bis 3, wobei das Volumen des Druckbehälters etwa 5 l oder größer, etwa 10 l oder größer oder etwa 50 l oder größer ist.

5. Das Verfahren gemäß einem der Ansprüche 1 bis 4, wobei das Protein ein therapeutisches Protein ist, das eine Antigenbindungsregion oder eine Antikörper-Fc-Region umfasst.

6. Das Verfahren gemäß einem der Ansprüche 1 bis 5, wobei die Mehrheit der Einschlusskörperpartikel in der Dispersion eine Absetzgeschwindigkeit von etwa 1 cm pro Stunde oder weniger hat.

7. Das Verfahren gemäß Anspruch 6, wobei die Mehrheit der Einschlusskörperpartikel, bezogen auf die Masse, eine Größe von 3 $\mu$m oder weniger oder etwa 2,2 $\mu$m oder weniger haben.

8. Das Verfahren gemäß Anspruch 1, wobei die mechanische Scherung durch Hochdruckhomogenisierung, Mikrofluidizer-Prozessoren oder Prozessoren mit fester Öffnung oder Prozessoren mit konstantem Druck erzeugt wird.

9. Das Verfahren gemäß einem der Ansprüche 1 bis 8, wobei die Chemie der Dispersion durch Zugabe eines oder mehrerer nicht-denaturierender Detergenzien, Pufferagenzien, Salze, Rückfaltungs-Co-Agenzien, viskositätserhöhender Agenzien oder bevorzugt Ausschlussverbindungen eingestellt wird.

10. Das Verfahren gemäß Anspruch 9, wobei das Zeta-Potential der Partikel eingestellt wird, so dass es außerhalb des Bereichs von ± 10 mV, oder ± 20 mV, oder ± 30 mV liegt.

11. Das Verfahren gemäß einem der Ansprüche 9 bis 10, wobei eine bevorzugte ausschließende Verbindung in einer Konzentration zugegeben wird, die eine Ausflockung verhindert.

12. Das Verfahren gemäß Anspruch 11, wobei die Einschlusskörperdispersion dem Gefrieren/Auftauen ausgesetzt wird und nicht ausflockt.

13. Das Verfahren gemäß Anspruch 1 bis 12, wobei das Einschlusskörperprotein ein Fusionsprotein mit einer Protease-Spaltstelle ist und zusammen mit einer zur Protease-Spaltung ausreichenden Protease einem hohen Druck ausgesetzt wird.

14. Das Verfahren gemäß Anspruch 13, wobei die Protease Pestivirus-Protease ist.

15. Das Verfahren gemäß einem der Ansprüche 1 bis 14, wobei die Einschlusskörperpartikel im Wesentlichen homogen in der Größe sind und um nicht mehr als 5 $\mu$m variieren.

16. Das Verfahren gemäß einem der Ansprüche 1 bis 15, wobei die Dispersion ohne Solubilisation oder Ausfällung von Protein aus Einschlusskörpern erzeugt wird.

**Revendications**

1. Procédé de production de protéine à partir de corps d'inclusion, comprenant :

> a) la fourniture d'une préparation de protéine comprenant des particules de corps d'inclusion,
> b) la préparation d'une dispersion de corps d'inclusion par cisaillement mécanique de façon que les particules de corps d'inclusion aient une taille de moins de 10 $\mu$m, et
> c) l'exposition de la dispersion de corps d'inclusion à une pression élevée dans un récipient sous pression, pour ainsi désagréger et replier la protéine de corps d'inclusion.

2. Procédé selon la revendication 1, comprenant en outre la sélection d'une chimie de solution de repliement sur la base d'un ou de plusieurs parmi le pH, la force ionique et la constante diélectrique ; dans lequel, facultativement, l'axe horizontal du récipient sous pression est au moins deux fois l'axe vertical.

**3.** Procédé selon la revendication 1, dans lequel la dispersion de corps d'inclusion est non dénaturante, et dans lequel, facultativement, la dispersion de corps d'inclusion ne contient pas de chaotropes et/ou de détergent suffisants pour solubiliser les corps d'inclusion en l'absence d'une pression élevée.

**4.** Procédé selon l'une quelconque des revendications 1 à 3, dans lequel le volume du récipient sous pression est d'environ 5 L ou plus, d'environ 10 L ou plus, ou d'environ 50 L ou plus.

**5.** Procédé selon l'une quelconque des revendications 1 à 4, dans lequel la protéine est une protéine thérapeutique qui comprend une région de liaison à l'antigène ou une région Fc d'anticorps.

**6.** Procédé selon l'une quelconque des revendications 1 à 5, dans lequel la majorité des particules de corps d'inclusion dans la dispersion présente une vitesse de sédimentation d'environ 1 cm par heure ou moins.

**7.** Procédé selon la revendication 6, dans lequel la majorité des particules de corps d'inclusion en masse présente une taille de 3 $\mu$m ou moins, ou d'environ 2,2 $\mu$m ou moins.

**8.** Procédé selon la revendication 1, dans lequel le cisaillement mécanique est généré par une homogénéisation à pression élevée, des processeurs de microfluidiseur ou des processeurs à orifice fixe ou à pression constante.

**9.** Procédé selon l'une quelconque des revendications 1 à 8, dans lequel la chimie de la dispersion est ajustée par l'ajout d'un ou de plusieurs parmi un détergent non dénaturant, un agent tampon, un sel, un co-agent de repliement, un agent augmentant la viscosité ou un composé d'exclusion préférentielle.

**10.** Procédé selon la revendication 9, dans lequel le potentiel zêta des particules est ajusté pour être hors de la plage de ± 10 mV, ou ± 20 mV, ou ± 30 mV.

**11.** Procédé selon l'une quelconque des revendications 9 à 10, dans lequel un composé d'exclusion préférentielle est ajouté à une concentration qui empêche la floculation.

**12.** Procédé selon la revendication 11, dans lequel la dispersion de corps d'inclusion est soumise à une congélation/décongélation et ne flocule pas.

**13.** Procédé selon l'une quelconque des revendications 1 à 12, dans lequel la protéine de corps d'inclusion est une protéine de fusion présentant un site de clivage de protéase, et est soumise à une pression élevée avec une protéase suffisante pour un clivage par protéase.

**14.** Procédé selon la revendication 13, dans lequel la protéase est une protéase de pestivirus.

**15.** Procédé selon l'une quelconque des revendications 1 à 14, dans lequel les particules de corps d'inclusion ont des tailles sensiblement homogènes, et ne varient pas de plus de 5 $\mu$m.

**16.** Procédé selon l'une quelconque des revendications 1 à 15, dans lequel la dispersion est créée sous solubiliser ou précipiter de protéine à partir des corps d'inclusion.

FIGURE 1

Particle Size of Representive Inclusion Body Preparations Prior to High Pressure Homogenization

FIGURE 2

FlowCAM Particle Sizing of rfG-CSF Inclusion Bodies

FIGURE 3

Calculated Inclusion Body Settling Distance (cm) that Occurs in 30 Minutes as a Function of Particle Size

FIGURE 4

FIGURE 5

FIGURE 6

FIGURE 7

**20% Glycerol Prevents Flocculation of Fab 1664 Inclusion Bodies During Freeze/Thaw**

FIGURE 8

**Effect of Dispersion Equipment on Fab 1664 Particle Size**

Legend: Agglomerated — Sonicated ····· Niro - - -

Y-axis: Mass Percent (%)

X-axis: Particle Size (μm) - Log Scale

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009208453 A
- US 7064192 B
- US 6489450 B
- US 20110268773 A
- US 8273561 B
- US 7767795 B
- US 20090215998 A
- US 20040038333
- WO 02062827 A
- WO 2007062174 A
- US 7829681 B

**Non-patent literature cited in the description**

- **ZHANG et al.** Modeling of protein refolding from inclusion bodies. *Acta Biochim. Biophys Sin,* 2009, 1044-1052
- **PETERNEL ; KOMEL.** Isolation of biologically active nanomaterial (inclusion bodies) from bacterial cells. *Microbial Cell Factories,* 2010, vol. 9, 66
- **BALDUINO et al.** Refolding by High Pressure of a Toxin Containing Seven Disulfide Bonds: Bothropstoxin-1 from Bothrops jararacussu. *Mol. Biotechnol.,* 2011, vol. 48, 228-234
- **VAN HEE et al.** Relation between cell disruption conditions, cell debris particle size, and inclusion body release. *Biotechnol. Bioeng. 5,* vol. 88 (1), 100-10
- **MALHOTRA ; COUPLAND.** The effect of surfactants on the solubility, zeta potential, and viscosity of soy protein isolates. *Food Hydrocolloids,* 2004, vol. 18, 101-108
- Handbook of Size Exclusion Chromatography and Related Techniques. Marcel Dekker, 2004, 439-462
- Size Exclusion Chromatography of Proteins. **BAKER et al.** Column Handbook for Size Exclusion Chromatography. Academic Press, 1999
- **SEEFELDT et al.** *Protein Sci,* 2005, vol. 14, 2258
- **KIM et al.** *J. Biol. Chem.,* 2001, vol. 276, 1626
- **KIM et al.** *J. Biol. Chem.,* 2002, vol. 277, 27240
- **HERMELING et al.** *Phar. Sci.,* 2006, vol. 95, 1084-1096
- **KILIC et al.** *Protein Sci,* 2003, vol. 12, 1663
- **WESTERMEIER, R.** Electrophoresis in Practice: A Guide to Methods and Applications of DNA and Protein Separations. John Wiley & Sons, 2005
- Gel Electrophoresis of Proteins: A Practical Approach. Oxford University Press, 1998
- **ARAKAWA, T. ; S. N. TIMASHEFF.** Stabilization Of Protein-Structure By Sugars. *Biochemistry,* 1982, vol. 21 (25), 6536-6544
- **BOWDEN, G. A. ; A. M. PAREDES et al.** Structure and morphology of protein inclusion-bodies in escherichia coli. *Biotechnology,* 1991, vol. 9 (8), 725-730
- **NEVERS, N.** Fluid Mechanics for Chemical Engineers. McGraw Hill, 1970
- **DUFFY, D. ; A. HILL.** *Suspension Stability: Why Particle Size, Zeta Potential and Rheology are Important,* 2011, www.malvern.comf.
- **LAIDLER, K. ; J. MEISER, EDS.** Physical Chemistry. Houghton Mifflin Company, 1995
- **QORONFLEH, W. ; L. K. HESTERBERG et al.** Confronting high-throughput protein refolding using high pressure and solution screens. *Protein Expression and Purification,* 2007, vol. 55, 209-224
- **SEEFELDT, M. B. ; C. CROUCH et al.** Specific volume and adiabatic compressibility measurements of native and aggregated recombinant human interleukin-1 receptor antagonist: Density differences enable pressure-modulated refolding. *Biotechnology And Bioengineering,* 2007, vol. 98 (2), 476-485
- **SEEFELDT, M. B. ; J. OUYANG et al.** High-pressure refolding of bikunin: Efficacy and thermodynamics. *Protein Science,* 2004, vol. 13 (10), 2639-2650
- **ST. JOHN, R. J. ; J. F. CARPENTER et al.** High pressure refolding of recombinant human growth hormone from insoluble aggregates - Structural transformations, kinetic barriers, and energetics. *Journal of Biological Chemistry,* 2001, vol. 276 (50), 46856-46863
- **ST. JOHN, R. J. ; J. F. CARPENTER et al.** High pressure fosters protein refolding from aggregates at high concentrations. *Proceedings of the National Academy of Sciences of the United States of America,* 1999, vol. 96 (23), 13029-13033

- **THOMAS, J. C. ; A. P. J. MIDDELBERG et al.** Sizing Biological Samples by Photosedimentation Techniques. *Biotechnology Progress,* 1990, vol. 6, 255-261
- **TIMASHEFF, S. N.** Water as Ligand - Preferential Binding and Exclusion of Denaturants in Protein Unfolding. *Biochemistry,* 1992, vol. 31 (41), 9857-9864
- **TIMASHEFF, S. N.** The Control of Protein Stability and Association by Weak-Interactions with Water - How Do Solvents Affect These Processes. *Annual Review of Biophysics and Biomolecular Structure,* 1993, vol. 22, 67-97